(19) Europäisches Patentamt
European Patent Office
Office européen des brevets

(11) **EP 3 719 251 A1**

(12) **DEMANDE DE BREVET EUROPEEN**

(43) Date de publication:
**07.10.2020 Bulletin 2020/41**

(51) Int Cl.:
*E21B 43/00* *(2006.01)*    *G01V 99/00* *(2009.01)*

(21) Numéro de dépôt: **20165435.7**

(22) Date de dépôt: **25.03.2020**

(84) Etats contractants désignés:
**AL AT BE BG CH CY CZ DE DK EE ES FI FR GB GR HR HU IE IS IT LI LT LU LV MC MK MT NL NO PL PT RO RS SE SI SK SM TR**
Etats d'extension désignés:
**BA ME**
Etats de validation désignés:
**KH MA MD TN**

(30) Priorité: **04.04.2019 FR 1903635**

(71) Demandeur: **IFP Energies nouvelles**
**92852 Rueil-Malmaison Cedex (FR)**

(72) Inventeurs:
• **NIETO DRAGHI, Carlos**
**92852 RUEIL-MALMAISON CEDEX (FR)**
• **DING, Didier Yu**
**92500 RUEIL-MALMAISON CEDEX (FR)**
• **SOBECKI, Nicolas**
**92852 RUEIL-MALMAISON CEDEX (FR)**
• **WU, Yu Shu**
**82852 RUEIL-MALMAISON CEDEX (FR)**

(54) **PROCEDE POUR EXPLOITER UN RESERVOIR PETROLIER FRACTURE AYANT UNE TAILLE DE PORES HETEROGENE**

(57)    L'invention concerne un procédé pour simuler des écoulements dans un réservoir géologique ayant une taille de pores hétérogène. A partir de mesures de laboratoire sur des échantillons prélevés dans le réservoir géologique, on détermine des classes de distribution de tailles de pores, et on détermine un modèle triple porosité représentatif de chacune des classes. Le modèle triple porosité selon l'invention comprend un milieu représentatif des pores de plus petite taille, un milieu représentatif des pores de plus grande taille, et un milieu représentatif des fractures. Le simulateur d'écoulement selon l'invention implémente le modèle triple porosité, une équation d'état thermodynamique prenant en compte une dimension équivalente des pores du milieu petits pores, des échanges de fluide exclusivement entre les milieux grands pores et petits pores et entre les milieux petits pores et fracture, et tient compte de la pression capillaire fonction de la saturation dans le milieu petits pores.

[Fig 1a]

EP 3 719 251 A1

## Description

### Domaine technique

La présente invention concerne le domaine de l'exploration et de l'exploitation réservoirs pétroliers ou de sites de stockage géologique de gaz.

Plus particulièrement, la présente invention concerne l'exploitation de réservoirs d'hydrocarbures non-conventionnels de très faible perméabilité (connus sous les termes anglais "shale gaz plays" et/ou "tight oil plays").

De manière générale, l'exploration et l'exploitation de réservoirs géologiques pétroliers nécessitent d'acquérir une connaissance aussi précise que possible de la géologie souterraine, et ce, afin de fournir de façon efficace une évaluation des réserves, une modélisation de la production, ou de la gestion de l'exploitation. En effet, la détermination de l'emplacement d'un puits de production et/ou d'un puits d'injection au sein d'un gisement d'hydrocarbures, la constitution de la boue de forage, les caractéristiques de complétion, le choix d'un procédé de récupération des hydrocarbures (tel que l'injection d'eau par exemple) et des paramètres nécessaires à la mise en oeuvre de ce procédé (tels que la pression d'injection, le débit de production,...) nécessitent de bien connaître le gisement. La connaissance d'un gisement signifie disposer d'une description aussi précise que possible de la structure, des propriétés pétrophysiques, des propriétés des fluides, etc, du gisement étudié.

Pour acquérir cette connaissance, l'industrie pétrolière allie les mesures sur champ (réalisées in situ, lors de campagnes sismiques, de mesures dans des puits, de carottages etc.) aux modélisations expérimentales (réalisées au laboratoire) ainsi qu'aux simulations numériques (réalisées au moyen de logiciels). La formalisation de cette connaissance passe ensuite par l'établissement d'une maquette du sous-sol, connue sous le terme de modèle géologique, qui permet de rendre compte de ces aspects de façon approchée. Généralement, ce type de maquette est représenté sur un ordinateur, et l'on parle alors de modèle numérique. Un modèle géologique a généralement une taille de mailles de l'ordre de la dizaine de mètres.

Afin de reproduire ou prédire (i.e. "simuler") la production d'hydrocarbures réelle, le spécialiste en ingénierie de réservoir met en œuvre sur ordinateur un logiciel de simulation d'écoulement, appelé également simulateur de réservoir. Le simulateur de réservoir calcule les écoulements et l'évolution des pressions au sein du réservoir représenté par un modèle de réservoir. Si la puissance informatique disponible pour réaliser les simulations d'écoulement le permet, le modèle de réservoir peut se confondre avec le modèle géologique. Dans le cas contraire, le modèle de réservoir peut être obtenu à l'issue d'une technique d'upscaling (mise à l'échelle), qui permet de passer du modèle géologique (modèle aux mailles plus fines) au modèle réservoir (modèle aux mailles plus grossières). Cette étape d'upscaling est bien connue du spécialiste en ingénierie de réservoir et peut être réalisée par exemple à l'aide du logiciel CobraFlow™ (IFP Energies nouvelles, France). Un modèle de réservoir a généralement une taille de mailles de l'ordre de la centaine de mètres. Ainsi, de manière générale, un simulateur d'écoulement calcule l'évolution spatiale et temporelle de l'écoulement et de la thermodynamique des fluides contenus dans un gisement ainsi que la production aux puits de production implantés dans ce gisement.

La demande mondiale en hydrocarbures augmentant, le développement de gisements non conventionnels à très faible perméabilité a éveillé l'intérêt des industriels du domaine pétrolier depuis plusieurs années comme une alternative aux gisements d'hydrocarbures conventionnels exploités jusque-là.

Or les écoulements et la thermodynamique des fluides d'hydrocarbures dans un gisement non conventionnel à très faible perméabilité sont beaucoup plus complexes que dans les gisements conventionnels sur plusieurs aspects. Tout d'abord la perméabilité de la matrice poreuse y est très faible, de l'ordre de quelques dizaines de nano-Darcy. La matrice poreuse doit donc être stimulée par fracturation hydraulique multi-étages afin de générer un milieu poreux fracturé très hétérogène, où l'écoulement se produit entre une matrice poreuse très peu perméable et les fractures jusqu'aux puits. La très faible perméabilité de la matrice implique un régime transitoire d'écoulement matrice/fractures très lent. De plus contrairement à une taille habituelle des pores d'un gisement conventionnel de l'ordre du micromètre, les pores des gisements à très faible perméabilité sont de l'ordre du nanomètre. La taille des molécules d'un hydrocarbure étant comprise entre 0.5 et 10nm, les forces d'interaction moléculaires de type Van der Waals entre le fluide et la paroi d'un pore nanométrique deviennent aussi importantes que les forces d'interaction entre molécules fluides. Le comportement thermodynamique du fluide est donc fortement modifié, les propriétés PVT (pression-volume-température) du fluide à l'équilibre liquide/vapeur ainsi que le point de bulle diffèrent fortement d'un cas où le fluide est non confiné et deviennent dépendants de la taille des pores. Enfin, la distribution de taille de pores dans un réservoir à très faible perméabilité est très hétérogène, passant du nanomètre au millimètre, ce qui provoque une très importante hétérogénéité spatiale de la pression capillaire et du comportement thermodynamique au sein de la matrice poreuse.

### Technique antérieure

Les documents suivants seront cités au cours de la description :

Alfi, M., An, C., Cao, Y. et al. 2017. Pore Size Variability and Sieving Effect in Liquid Shale-A Multiple Permeability Approach and Eagle Ford Case Study. SPE Reservoir Simulation Conference, 20-22 February, Montgomery, Texas, USA. https://doi.org/10.2118/182643-MS.

E.P. Barret, L.G. Joyner, P.B. Halenda J. Am. Chem. Soc., 73 (1951), p. 373.

Bird, Robert Byron; Stewart, Warren E.; Lightfoot, Edwin N. (2007) Transport phenomena. Revised 2nd édition. New York etc. : John Wiley & Sons Inc (1).

Chalmers, Gareth R.; Bustin, R. Marc; Power, Ian M. (2012) Characterization of gas shale pore systems by porosimetry, pycnometry, surface area, and field emission scanning electron microscopy/transmission electron microscopy image analyses. Examples from the Barnett, Woodford, Haynesville, Marcellus, and Doig units. In : AAPG Bulletin, vol. 96, n°6, p. 1099-1119. DOI: 10.1306/10171111052.

Jin, Luchao; Ma, Yixin; Jamili, Ahmad (2013) Investigating The Effect of Pore Proximity on Phase Behavior And Fluid Properties in Shale Formations. SPE Annual Technical Conférence and Exhibition. Richardson, TX.

Kuila, Utpalendu; Prasad, Manika (2011) Understanding Pore-Structure And Permeability In Shales: SPE Annual Technical Conférence and Exhibition, 30 October-2 November, Denver, Colorado, USA. https://doi.org/10.2118/146869-MS

Kuila, Utpalendu; Prasad, Manika (2013) Specific surface area and pore-size distribution in clays and shales. In : Geophysical Prospecting, vol. 61, n° 2, p. 341-362. DOI: 10.1111/1365-2478.12028.

Le Ravalec M, Noetinger B, Hu LY (2002) The FFT moving average (FFT-MA) generator: an efficient tool for generating and conditioning Gaussian simulations. Math Geol 32(6):701-723.

Meyra, Ariel G.; Zarragoicoechea, Guillermo J.; Kuz, Victor A. (2005) Thermodynamic equations for a confined fluid at nanometric scale. In : Fluid Phase Equilibria, vol. 230, n° 1-2, p. 9-14. DOI: 10.1016/j.fluid.2004.10.014.

Pruess, K. (1985). A Practical Method for Modeling Fluid and Heat Flow in Fractured Porous Media. Society of Petroleum Engineers. doi:10.2118/10509-PA.

Warren, J.E. et Root, P.J., "The Behavior of Naturally Fractured Reservoirs", SPE Journal (septembre 1963), 245-255.

Il est classique d'utiliser des approches de type "double milieu" ou encore "double porosité" pour représenter la complexité des milieux fracturés. Décrite par exemple dans (Warren et Root, 1963), cette approche suppose que tout volume élémentaire (maille du modèle de réservoir) du réservoir fracturé est modélisé sous la forme d'un ensemble de blocs parallélépipédiques identiques, appelés blocs matriciels, délimités par un système orthogonal de fractures uniformes continues orientées suivant les directions principales d'écoulement. L'écoulement des fluides, à l'échelle du réservoir, s'effectue à travers les fractures pour l'essentiel, et des échanges de fluides interviennent localement entre les blocs matriciels et les fractures . Le plus souvent, les mailles ont des dimensions latérales hectométriques (couramment 100 ou 200 m) compte tenu de la taille des champs et des possibilités limitées des logiciels de simulation en termes de capacité et temps de calcul. Il en résulte que, pour la plupart des champs fracturés, le volume élémentaire (maille) de réservoir fracturé renferme d'innombrables fractures formant un réseau complexe délimitant de multiples blocs matriciels de dimensions et formes variables suivant le contexte géologique. Il est alors possible de formuler et calculer les flux d'échange matrice-fissure pour ce bloc matriciel représentatif, et d'en multiplier le résultat par le nombre de tels blocs dans le volume élémentaire (maille) pour obtenir le flux à l'échelle de cette maille.

Ce type de représentation double porosité n'est toutefois pas adaptée dans le cas de réservoirs à très faible perméabilité ayant subi par exemple une stimulation par fracturation hydraulique. En effet, un tel modèle ne prend pas en compte le régime transitoire très long de l'écoulement matrice/fracture dû à la très faible perméabilité de la matrice. De plus, ce type de modèle ne prend pas en compte la thermodynamique du fluide dépendant de la taille du pore.

On connait en outre le document (Alfi et al. 2017) qui décrit un modèle dit "triple porosité", dans lequel le milieu fracturé est divisé en un milieu représentatif des fractures, un milieu représentatif des plus grands pores du milieu matriciel et un milieu représentatif des plus petits pores du milieu matriciel. Autrement dit, par rapport au modèle double porosité, le modèle triple porosité selon (Alfi et al. 2017) scinde le milieu matriciel en deux sous-milieux, un milieu représentatif des pores de la matrice poreuse de plus grande taille, et un milieu représentatif des pores de la matrice poreuse de plus petite taille. Par ailleurs, le modèle décrit dans ce document implémente une équation d'état thermodynamique classique, telle que de Peng Robinson pour le milieu "grands pores" et le milieu "fractures", et une équation d'état thermodynamique telle que décrite dans (Travalloni et al. 2014) pour le milieu "petit pores". L'équilibre thermodynamique entre petits pores et grands pores est vérifié. L'écoulement est possible entre tous les milieux et aucune discrétisation du milieu matriciel n'est effectuée. Le régime transitoire très lent entre matrice et fracture n'est donc pas pris en compte. De plus, aucune vérification du modèle à l'aide d'une simulation à l'échelle fine n'est réalisée.

Ainsi, la présente invention vise à améliorer la simulation des écoulements dans le cas d'un réservoir géologique caractérisé par une distribution hétérogène de la taille des pores, variant du nanomètre au millimètre, et ce en vue d'une meilleure prédiction de la production du fluide contenu dans ce réservoir. Plus précisément, la présente invention concerne un procédé pour simuler les écoulements d'un fluide contenu dans un réservoir géologique présentant une grande hétérogénéité dans la tailles des pores, la simulation des écoulements étant réalisée au moyen d'un simulateur d'écoulement implémentant au moins un modèle triple porosité, et permettant la prise en compte du régime transitoire très lent entre matrice poreuse et fractures, de la thermodynamique dépendant de la taille des pores ainsi que la prise en compte d'hétérogénéités de pression capillaire.

En outre, l'invention concerne un procédé d'exploitation d'un fluide contenu dans un réservoir géologique fracturé caractérisé par une distribution hétérogène de la taille des pores, mettant en œuvre un procédé de simulation des écoulements tel que décrit ci-dessous, pour prédire des courbes de production de ce fluide dans le temps en fonction de différents schéma d'exploitation de ce réservoir, et ainsi déterminer un schéma d'exploitation optimal pour produire ce fluide.

**Résumé de l'invention**

La présente invention concerne un procédé mis en œuvre par ordinateur pour simuler des écoulements d'un fluide, ledit fluide comprenant une phase huile et une phase gazeuse, dans un réservoir géologique fracturé présentant une taille de pores hétérogène, dans lequel, on construit une première représentation maillée dudit réservoir à partir de mesures de propriétés relatives audit réservoir. Le procédé selon l'invention comprend au moins les étapes suivantes:

A) A partir de mesures réalisées en laboratoire sur une pluralité d'échantillons provenant de différentes positions spatiales dans ledit réservoir géologique, on détermine des classes de distribution de tailles de pores, et on attribue une classe de distribution de tailles de pores en chacune des mailles de ladite première représentation maillée ;
B) pour chacune desdites classes de distribution de tailles de pores, on construit un modèle de porosité représentatif de ladite distribution de tailles de pores, ledit modèle de porosité comprenant un premier milieu représentatif des pores de ladite distribution dont la dimension est comprise dans une première gamme, un deuxième milieu représentatif des pores de ladite distribution dont la dimension est comprise dans une deuxième gamme, et un troisième milieu représentatif desdites fractures de ladite distribution, lesdites dimensions desdits pores de ladite première gamme étant supérieures aux dimensions desdits pores de ladite deuxième gamme, ledit modèle de porosité étant en outre décrit par des paramètres d'écoulement pour chacun desdits milieux, lesdits paramètres d'écoulement comprenant au moins une dimension équivalente desdits pores dudit deuxième milieu et une pression capillaire fonction d'une saturation en ladite phase gazeuse dudit fluide dans ledit second milieu ;
C) pour chacune desdites classes de distribution de tailles de pores, on cale au moins une partie desdits paramètres dudit modèle de porosité représentatif de ladite distribution de tailles de pores et on attribue ledit modèle de porosité calé en chacune des mailles de ladite première représentation maillée à laquelle est attribuée ladite classe ;

Puis, selon l'invention, on simule lesdits écoulements dudit fluide dans ledit réservoir géologique au moyen de ladite première représentation maillée et d'un premier simulateur d'écoulement, ledit premier simulateur implémentant au moins :

- lesdits modèles de porosité calés et attribués en chacune des mailles de ladite première représentation maillée ;
- pour ledit second milieu, une équation d'état thermodynamique prenant en compte ladite dimension équivalente desdits pores dudit second milieu ;
- au sein desdits modèles de porosité, des échanges dudit fluide entre ledit premier milieu et ledit deuxième milieu et entre ledit deuxième milieu et ledit troisième milieu d'un même modèle de porosité, en tenant compte de ladite pression capillaire fonction de ladite saturation en ladite phase gazeuse dudit fluide dans ledit second milieu ;
- entre au moins deux desdits modèles de porosité, des échanges dudit fluide exclusivement entre lesdits troisièmes milieux desdits deux modèles de porosité.

Selon une mise en œuvre de l'invention, ledit calage pour une desdites classes de distribution de pores peut être réalisé selon les étapes suivantes :

a) on simule les écoulements au moyen dudit premier simulateur et dudit modèle de porosité représentatif de ladite classe de distribution de tailles de pores, ledit modèle de porosité étant défini par lesdits paramètres d'écoulement dudit modèle, et on obtient des courbes de production fonction desdits paramètres d'écoulement dudit modèle de porosité ;
b) on mesure les écarts entre lesdites courbes de production fonction desdits paramètres d'écoulement dudit modèle de porosité et des courbes de production de référence prédéterminées, et on corrige au moins une partie desdits paramètres d'écoulement dudit modèle de porosité de manière à minimiser lesdits écarts ;
c) on réitère les étapes a) et b) jusqu'à ce que lesdits écarts soient inférieurs à un seuil prédéfini, lesdits paramètres d'écoulement dudit modèle de porosité pour une itération de ladite réitération correspondant auxdits paramètres corrigés à une itération précédente de ladite réitération.

Selon une mise en œuvre de l'invention, on peut déterminer lesdites courbes de référence selon les étapes suivantes:

i) on construit une deuxième représentation maillée représentative de l'hétérogénéité de la taille des pores de ladite classe de distribution de tailles de pores, les dimensions des mailles de ladite deuxième représentation maillée étant déterminées de manière à rendre compte des effets induits par ladite taille des pores de ladite distribution ;
ii) on simule des écoulements au moyen de ladite deuxième représentation maillée et d'un deuxième simulateur d'écoulement implémentant au moins une équation d'état thermodynamique prenant en compte lesdites dimensions desdits pores de ladite classe de distribution de tailles pores, et on obtient des courbes de production dudit fluide relatives à ladite deuxième représentation maillée.

Selon une mise en œuvre de l'invention, ladite partie desdits paramètres d'écoulement corrigés peut comprendre ladite dimension équivalente desdits pores dudit deuxième milieu, une perméabilité et une porosité équivalentes dudit deuxième milieu ou une perméabilité et une porosité équivalentes dudit premier milieu, et un paramètre représentatif de la proportionnalité de la transmissibilité dudit premier milieu audit deuxième milieu. Avantageusement, lesdites mesures en laboratoire peuvent être réalisées au moyen d'une méthode de porosimétrie au mercure et une méthode d'adsorption/désorption par azote. Selon une mise en œuvre de l'invention, on peut discrétiser lesdits premier milieu et deuxième milieux par une discrétisation de type couronnes emboîtées.
En outre, l'invention concerne un produit programme d'ordinateur téléchargeable depuis un réseau de communication et/ou enregistré sur un support lisible par ordinateur et/ou exécutable par un processeur, comprenant des instructions de code de programme pour la mise en œuvre du procédé tel que décrit ci-dessus, lorsque ledit programme est exécuté sur un ordinateur.
L'invention concerne également un procédé pour exploiter un fluide contenu dans un réservoir géologique fracturé présentant une taille de pores hétérogène, dans lequel on applique le procédé mis en œuvre par ordinateur pour simuler des écoulements d'un fluide tel que décrit ci-dessus, et dans lequel, à partir au moins de ladite simulation desdits écoulements dans ledit réservoir géologique, on détermine un schéma d'exploitation dudit réservoir géologique comprenant au moins une implantation d'au moins un puits injecteur et/ou d'au moins un puits producteur, et on exploite ledit fluide dudit réservoir géologique au moins en forant lesdits puits de ladite implantation et en les équipant d'infrastructures d'exploitation.
D'autres caractéristiques et avantages du procédé selon l'invention, apparaîtront à la lecture de la description ci-après d'exemples non limitatifs de réalisations, en se référant aux figures annexées et décrites ci-après.

## Liste des figures

Les figures 1a et 1b illustrent une section selon un plan horizontal dans une répartition des perméabilités équivalentes du milieu fractures d'un réservoir géologique et dans une répartition de 5 classes de distribution de tailles de pores du milieu matriciel de ce réservoir géologique.

La figure 2 illustre une section selon un plan horizontal dans une représentation maillée à l'échelle fine d'une distribution de tailles de pores.

Les figures 3a à 3c comparent des courbes de production de référence avec des courbes de production obtenues pour un exemple de classe de distribution de pores, après calage selon l'étape 3 du procédé selon l'invention.

Les figures 4a à 4c comparent des courbes de production obtenues au moyen du procédé selon l'invention, au moyen d'un procédé selon l'art antérieur, et par des mesures in situ.

Les figures 5a à 5i illustrent des champs de pression huile, des champs de pression gaz et champs de saturation en gaz pour chacun des milieux du modèle triple porosité selon l'invention.

## Description des modes de réalisation

De façon générale, le premier aspect de l'invention concerne un procédé pour simuler des écoulements d'un fluide dans un réservoir géologique fracturé présentant une taille de pores hétérogène. Par taille de pores hétérogène, on entend une taille de pores variant de manière générale du nanomètre au millimètre. Le fluide contenu dans le réservoir géologique étudié comprend au moins une phase huile et une phase gazeuse.

Selon un deuxième aspect, l'invention concerne un procédé pour exploiter un fluide contenu dans un réservoir géologique fracturé présentant une taille de pores hétérogène, au moyen d'un procédé pour simuler les écoulements d'un fluide dans un réservoir géologique selon le premier aspect de l'invention.

La présente invention requiert de disposer :

- de mesures de propriétés relatives au réservoir géologique étudié : il s'agit d'une part de mesures de propriétés pétrophysiques, réalisées in situ ou bien en laboratoire, telles que des mesures de la porosité, la perméabilité, la lithologie (c'est-à-dire le type de roche), la perméabilité relative ou encore la pression capillaire. Ces mesures peuvent avoir été obtenues par exemple par analyse en laboratoire de carottes prélevées in situ, ou bien via des diagraphies réalisées dans des puits traversant le réservoir étudié, ou encore par des campagnes d'acquisition sismique. Il s'agit d'autre part de mesures des propriétés des fluides s'écoulant dans le réservoir géologique étudié, telles que des mesures de débits d'huile, de débits d'eau, de pression ou encore de saturation. Ces mesures peuvent avoir été obtenues par exemple par des mises en production du fluide en certains puits traversant le réservoir géologique étudié, au cours de tests de puits ou encore de tests d'interférence. De manière générale, il s'agit de mesures de propriétés bien connues par le spécialiste en simulation d'écoulement dans un réservoir géologique ;
- d'une représentation maillée représentative du réservoir géologique étudié : appelée aussi modèle de réservoir, il s'agit d'une sorte de maquette du sous-sol construite dans le but de décrire aussi précisément que possible la structure, les propriétés pétrophysiques et les propriétés des fluides du réservoir étudié. Cette maquette est généralement représentée sur un ordinateur, et consiste en un maillage ou grille, chacune des mailles de cette grille comportant une ou plusieurs valeurs de propriétés relatives au réservoir étudié (telles que porosité, perméabilité, saturation, faciès géologique, pression etc). Un modèle de réservoir se doit de vérifier autant que possible les propriétés collectées sur le terrain : les données de diagraphie mesurées le long des puits, les mesures réalisées sur des échantillons de roche prélevés par exemple par carottage, les données déduites de campagnes d'acquisition sismique, les données de production comme les débits d'huile, d'eau, les variations de pression etc. Le spécialiste en simulation de réservoir a pleine connaissance de méthodes pour construire une telle représentation maillée d'un réservoir géologique. A noter que le modèle de réservoir peut se confondre avec le modèle géologique lorsque la puissance informatique est suffisante pour permettre des calculs numériques de simulation d'écoulement sur une grille à mailles fines. Dans les autres cas, le spécialiste pourra avoir recours à une technique d'« upscaling » (mise à l'échelle) afin de passer d'un modèle aux mailles fines (le modèle géologique) à un modèle aux mailles plus grossières (le modèle de réservoir). Cette étape d'upscaling peut être réalisée par exemple à l'aide du logiciel CobraFlow™ (IFP Energies nouvelles, France). Selon une mise en œuvre de l'invention, la représentation maillée du réservoir géologique étudié comporte au moins une valeur de saturation, une valeur de pression, une température, une composition du mélange fluide, une perméabilité, une porosité, une pression capillaire, une perméabilité relative et une taille de pores dans chacune de ses mailles.
- d'un simulateur d'écoulement selon l'invention : un simulateur d'écoulement est un programme numérique, exécuté sur un ordinateur, qui est utilisé pour simuler l'écoulement de fluides, dans le cas d'espèce, dans un réservoir géologique. La simulation d'écoulement, dite aussi simulation de réservoir dans ce cas d'espèce, consiste à prédire numériquement la production au cours du temps d'un fluide piégé dans un réservoir géologique, la production nécessitant l'existence d'au moins un puits producteur (vers lequel le fluide piégé, va se déplacer par gradient de pression et duquel il va pouvoir être extrait). Le simulateur d'écoulement selon l'invention, qui implémente notamment un modèle triple porosité, sera décrit de manière détaillée dans l'étape 3 ci-dessous. Selon une mise en œuvre de l'invention, le simulateur selon l'invention peut en outre implémenter un modèle simple porosité, tel que cela sera décrit dans l'étape 4 ci-dessous.

Le procédé selon le premier aspect de l'invention comporte au moins les étapes 1 à 4 décrites ci-après. Le procédé selon le deuxième aspect de l'invention comporte au moins les étapes 1 à 5 décrites ci-après.

**1) Détermination de classes de distribution de tailles de pores**

Selon l'invention, on réalise des mesures en laboratoire sur une pluralité d'échantillons de roche prélevés en différentes positions spatiales (par exemple représentées par des positions (x,y,z) dans un repère géographique) du réservoir géologique étudié, et on détermine au moins une distribution de tailles de pores relative à chacun des échantillons à partir de ces mesures. Puis selon l'invention, à partir au moins de ces mesures, on détermine des classes de distribution de tailles de pores, et on attribue une classe de distribution de tailles de pores à chaque échantillon. Autrement dit, on regroupe par catégorie, ou encore par type, les différentes distributions de tailles de pores mesurées. Par exemple, si on réalise des mesures de distributions de tailles de pores sur 50 échantillons distincts, on peut déterminer des ressemblances entre ces 50 distributions de tailles de pores et les organiser par exemple en 5 classes. Selon une mise en œuvre de l'invention, on détermine de préférence au moins trois classes de distribution de tailles de pores. Une classe peut être identifiée par un chiffre, une lettre, une combinaison de chiffres, une combinaison de lettres ou tout autre identifiant.

Selon une mise en œuvre de l'invention, cette classification peut prendre en compte la porosité et la perméabilité associées à chacun des échantillons. Il peut s'agir de la porosité et de la perméabilité mesurées directement sur chacun des échantillons, ou bien de la porosité et de la perméabilité dans la maille de la représentation maillée du réservoir géologique à laquelle appartient cet échantillon. En effet, il y a un lien direct entre porosité et perméabilité d'une part et distribution de tailles de pores d'autre part, si bien qu'une classification appliquée sur la porosité et la perméabilité peut être avantageuse pour déterminer des classes de distribution de tailles de pores.

Selon une mise en œuvre préférée de l'invention, les mesures de laboratoire pour déterminer la distribution de tailles de pores d'un échantillon de roche du réservoir géologique comprennent la combinaison de la mise en œuvre de la méthode de mesure de porosimétrie au mercure ("Mercury Intrusion Porosimetry" en anglais, aussi connue sous l'acronyme "MIP") et de la méthode de mesure d'adsorption/désorption par azote ("Nitrogen gas adsorption" en anglais, aussi connue sous l'acronyme N2GA). La méthode de porosimètrie au mercure est une méthode classique pour déterminer une distribution de tailles de pores. Le mercure est un liquide non-mouillant qui ne pénètre pas spontanément dans les pores par capillarité, il est nécessaire de lui appliquer une pression. Le mercure va pénétrer sous l'effet d'une pression croissante dans des pores de plus en plus petits. La mesure du volume d'intrusion en fonction de la pression permet la détermination de la distribution de tailles de pores. En particulier, l'équation dite de Washburn permet de faire le lien entre pression et taille de pores. Cependant la méthode de porosimétrie au mercure n'est pas capable de prendre en compte les pores de très petite taille. Une méthode d'adsorption/désorption par Azote (N2) est donc avantageusement mise en œuvre en complément de la méthode MIP. A partir des isothermes d'adsorption et désorption, la distribution de tailles de pores peut être obtenue. Le modèle le plus couramment utilisé est celui décrit dans (Barret, et al., 1951). Ainsi, la combinaison de mesures par les méthodes MIP et N2GA est avantageuse car, la méthode MIP ne permettant pas de décrire la structure complète des pores les plus petits, la méthode N2GA vient en complément, notamment en étant capable de mesurer les pores d'un diamètre en deçà de 200 nm.

Selon une autre mise en œuvre de l'invention, on peut aussi utiliser d'autres méthodes appropriées pour déterminer une distribution de tailles de pores d'un échantillon de roche telle que décrites dans les documents (Kuila and Prasad, 2011 ; Kuila, and Prasad, 2013 ; Chalmers et al., 2012°).

Selon une mise en œuvre de l'invention, on réalise une classification de la pluralité de distributions de tailles de pores au moyen de la méthode de classification dite des K-means. De manière générale, l'algorithme des K-Means permet de regrouper les valeurs de variables en K classes ne se chevauchant pas. On choisit un nombre de classes (ou coefficient K), en général inférieur à 10, afin d'obtenir un résultat relativement stable. Cet algorithme présente les avantages d'une simplicité conceptuelle, d'une rapidité d'exécution et de faibles exigences en taille mémoire.

Selon une mise en œuvre de l'invention, après avoir déterminé des classes de distribution de tailles de pores à partir de la pluralité de mesures de laboratoire réalisées sur la pluralité d'échantillons de roche du réservoir géologique étudié, une classe de distribution de tailles de pores étant alors attribuée à chacun des échantillons, on attribue une classe en chacune des mailles de la représentation maillée. Selon une mise en œuvre de l'invention, à partir de la position spatiale dans le réservoir géologique étudié de chacun des échantillons, on en déduit la maille de la représentation maillée à laquelle ils appartiennent, on attribue la classe de distribution de tailles de pores déterminée pour chacun de ces échantillons dans leurs mailles respectives ainsi identifiées, puis par exemple au moyen d'une méthode géostatistique telle que décrite dans le document (Le Ravalec et al., 2002) on attribue une classe de distribution de tailles de pores dans chacune des mailles de la représentation maillée pour lesquelles aucune classe de distribution de tailles de pores n'a été attribuée. Cela peut être le cas pour des mailles dans lesquelles aucun échantillon de roche n'a été prélevé, et donc des mailles pour lesquelles on ne disposait pas d'une mesure directe de distribution de tailles de pores.

## 2) Construction d'un modèle triple porosité pour chaque classe de distribution de tailles de pores

La deuxième étape du procédé selon l'invention est appliquée pour chacune des classes de distribution de tailles de pores identifiées à l'issue de l'étape 1 décrite ci-dessus.

Selon l'invention, pour chaque classe de distribution de tailles de pores déterminée à l'étape 1, on construit un modèle dit triple porosité représentatif de la classe de distribution de tailles de pores considérée. Plus précisément, le modèle triple porosité selon l'invention est défini de la manière suivante : un premier milieu représentatif des pores de la distribution dont la dimension est comprise dans une première gamme, un deuxième milieu représentatif des pores de la distribution dont la dimension est comprise dans une deuxième gamme, et un troisième milieu représentatif des fractures, les dimensions des pores de la première gamme étant supérieure aux dimensions des pores de ladite deuxième gamme. Par la suite et à des fins de simplification de l'exposé, le premier milieu est dit "milieu grands pores", le deuxième milieu est dit "milieu petits pores" et le troisième milieu est dit "milieu fractures". Autrement dit et de manière simplifiée, on détermine deux gammes pour les tailles pores de chaque distribution de tailles de pores déterminée à l'étape 1: une première gamme dans laquelle les pores sont de plus grande taille que ceux de la deuxième gamme. Selon une mise en œuvre de l'invention, la première gamme du modèle triple porosité définit des pores ayant un diamètre par exemple supérieur ou égal à 100 nm, la deuxième gamme du modèle triple porosité selon l'invention définit des pores ayant un diamètre inférieur à 100 nm. Comparé à un modèle de type double milieu, pour lequel un bloc parallélépipédique représentatif de la matrice poreuse est délimité par un réseau orthogonal et régulier de fractures, le modèle triple porosité selon l'invention représente la matrice poreuse par deux blocs matriciels, l'un représentatif des petits pores de cette matrice et l'autre représentatif des grands pores de cette matrice.

Selon l'invention, on attribue en outre des paramètres d'écoulement pour chacun des trois milieux du modèle triple porosité ainsi défini. Selon l'invention, on attribue au moins au moins une dimension (par exemple le rayon) équivalente (ou autrement dit effective) des pores du milieu petits pores. Par ailleurs, selon l'invention, une pression capillaire fonction de la saturation en gaz est attribuée au milieu petits pores uniquement, et on attribue une pression capillaire valant zéro à la pression capillaire dans le milieu grands pores.

De manière implicite, les paramètres d'écoulement du modèle de porosité comprennent en outre une porosité et une perméabilité équivalentes (ou encore effectives) pour chaque milieu constituant le modèle de porosité, ainsi qu'une perméabilité relative et une pression capillaire pour chaque milieu constituant le modèle de porosité.

Selon une mise en œuvre de l'invention, les paramètres d'écoulement du modèle de porosité comprennent la dimension équivalente des pores du milieu petits pores, la perméabilité et la porosité équivalentes du milieu petits pores (ou de manière équivalente la perméabilité et la porosité équivalentes du milieu grands pores), et un paramètre $\alpha$ qui est représentatif de la proportionnalité de la transmissibilité du milieu grands pores au milieu petits pores. Selon cette mise en œuvre, la somme des perméabilités (respectivement porosités) petits pores et grands pores est égale à la perméabilité déterminée pour la classe de distribution de tailles de pores considérée. Tel que cela sera décrit à l'étape 3, on peut mettre à jour la répartition initiale entre la répartition des perméabilités (respectivement porosités) petits pores et grands pores par une méthode de calage.

Selon une mise en œuvre de l'invention, on peut utiliser la méthode décrite dans la demande de brevet EP3181804 (US 2017-0212276) pour déterminer la taille des blocs matriciels. De manière générale, on peut utiliser le logiciel FRACAFLOW® (IFP Energies nouvelles, France) pour déterminer des perméabilités et porosités équivalentes de fracture.

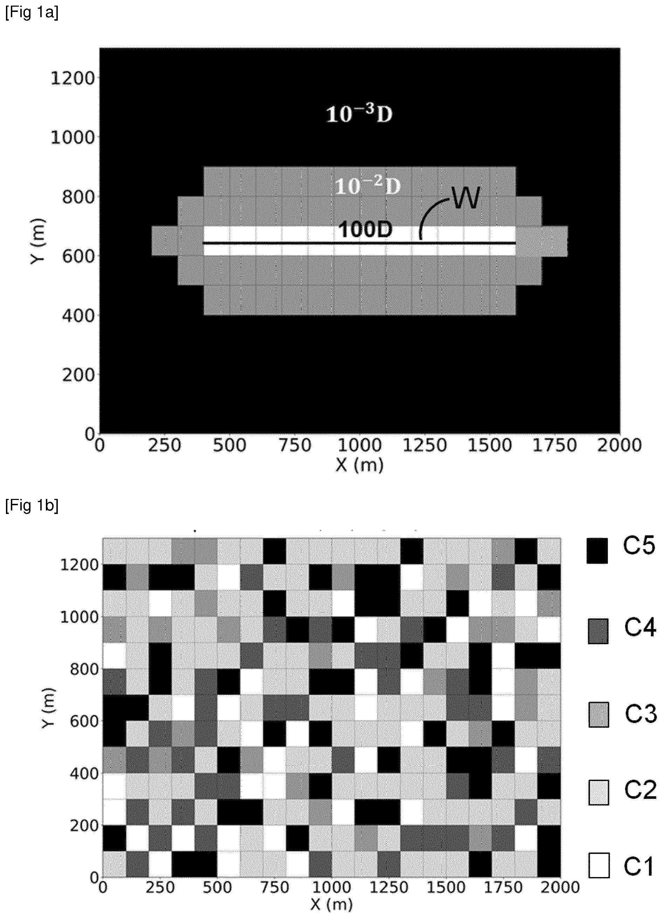

A titre illustratif, les figures 1a et 1b illustrent une section à z constant (ou autrement un plan horizontal) dans une représentation maillée d'un réservoir géologique montrant la répartition des perméabilités équivalentes dans le milieu fractures (figure 1a) et la répartition de 5 classes de distributions de tailles de pores notées C1 à C5 (identifiées chacune par une couleur propre ; figure 1b). On peut observer une perméabilité très forte (100 D) dans le milieu fractures le long du segment horizontal W représentatif d'un puits, et fortement décroissante (de $10^{-2}$ D à $10^{-3}$ D) quand on s'éloigne de ce puits. Il s'agit d'un puits de stimulation hydraulique du milieu fracturé, ce qui explique la très forte perméabilité de fractures proche du puits de fracturation.

**3) Calage d'au moins une partie des paramètres d'écoulement des modèles de porosité déterminés pour chacune des classes de distribution de tailles de pores**

La troisième étape du procédé selon l'invention est appliquée pour chaque classe de distribution de tailles de pores identifiée à l'étape 1 ci-dessus, et pour laquelle un modèle de porosité a été construit tel que décrit à l'étape 2 ci-dessus.

Selon l'invention, pour chacune des classes de distribution de tailles de pores, il s'agit de caler au moins une partie des paramètres d'écoulement du modèle de porosité construit pour cette classe de distribution de tailles de pores.

Selon une mise en œuvre de l'invention, pour une classe de distribution de tailles de pores donnée, on réalise un calage de paramètres d'écoulement de cette classe au moyen de courbes de production de référence prédéfinies, et selon les étapes suivantes :

a) on simule des écoulements au moyen du simulateur d'écoulement selon l'invention (cf. description détaillée du simulateur selon l'invention à l'étape 4 ci-dessous), appliqué au modèle de porosité représentatif de la classe de distribution de tailles de pores tel que construit à l'étape précédente, le modèle de porosité étant en outre décrit par des paramètres d'écoulement. On obtient des courbes de production relatives au modèle de porosité établi à l'étape 2, et donc fonction de paramètres d'écoulement du modèle de porosité ;

b) puis on mesure les écarts entre les courbes de production relatives au modèle de porosité et les courbes de production de référence, et on corrige les paramètres du modèle de porosité de manière à minimiser ces écarts ;

c) on réitère les étapes a) et b) jusqu'à ce que les écarts soient inférieurs à un seuil prédéfini, les paramètres du modèle de porosité utilisés pour une nouvelle itération de l'étape a) correspondant aux paramètres corrigés pour une itération précédente. Selon une mise en œuvre de l'invention, on peut utiliser un algorithme d'optimisation, par exemple basé sur la méthode du gradient conjugué, pour minimiser de manière automatisée et selon un processus itératif, une fonction objectif mesurant les écarts entre courbes de production de référence et courbes de production simulées pour des valeurs de paramètres d'écoulement à une itération donnée.

Selon une mise en œuvre de l'invention, on détermine des courbes de production de référence selon les étapes suivantes :

i) on construit une représentation maillée dite "à l'échelle fine", dont les dimensions des mailles sont suffisamment fines pour rendre compte des effets induits par la taille des pores de la distribution considérée sur les écoulements. De manière générale, on peut considérer qu'un telle représentation maillée est formée de mailles de dimension au moins 100 fois inférieures aux dimensions des mailles de la représentation maillée du réservoir géologique. Ainsi, il ne s'agit pas de construire une représentation maillée représentative du réservoir géologique en entier, mais uniquement représentative d'une portion du réservoir dans laquelle des échanges matrices/fractures ont lieu. Il s'agit donc d'une représentation maillée fictive, à l'échelle fine. Cette représentation maillée peut par exemple avoir des mailles de dimensions 0.2 m x 0.2 m x 0.2 m et peut avoir une dimension globale de 20 m x 20 m x 20 m.
ii) on simule des écoulements au moyen de cette représentation maillée à l'échelle fine et d'un simulateur d'écoulement implémentant un modèle simple porosité et au moins une équation d'état thermodynamique prenant en compte les dimensions des pores de la classe de distribution de tailles de pores. Selon une mise en œuvre de l'invention, l'équation d'état thermodynamique peut permettre une modification du point critique, ou l'équation d'état thermodynamique correspond à toute autre variante. On obtient de cette manière des courbes de production du fluide relatives à cette représentation maillée à l'échelle fine, et donc représentatives des écoulements à l'échelle fine. Ces courbes de production peuvent alors servir de courbes de référence pour le calage des paramètres d'écoulement du modèle de porosité selon cette mise en œuvre de l'invention.

Selon une mise en œuvre très préférée de l'invention, les paramètres d'écoulement qui sont mis à jour lors de cette étape sont : la dimension équivalente des pores du milieu petits pores, la perméabilité et la porosité équivalentes du milieu petits pores (ou de manière équivalente la perméabilité et la porosité équivalentes du milieu grands pores) et un paramètre $\alpha$ qui est représentatif de la proportionnalité de la transmissibilité du milieu grands pores au milieu petits pores. La dimension équivalente des pores du milieu petits pores est caractéristique de la distribution de tailles de pores et varie en fonction de la fraction de petits pores dans la matrice poreuse. La répartition de la perméabilité et porosité entre petits et grands pores est aussi caractéristique de la distribution de tailles de pores. Le paramètre $\alpha$ va dépendre du contact grands pores petits pores dans la simulation fine.

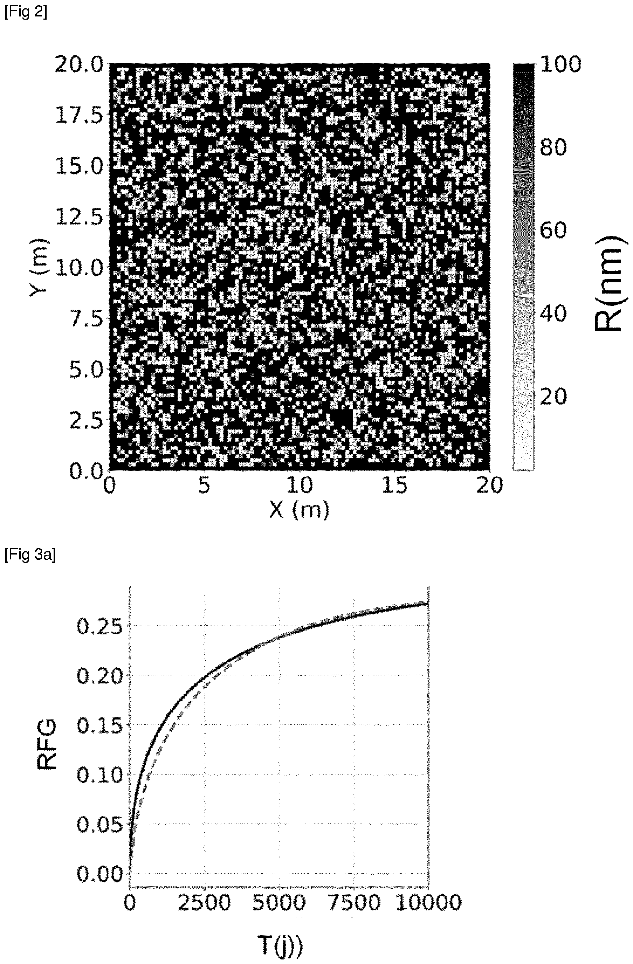

La figure 2 présente, à titre purement illustratif, une section selon un plan horizontal (dans un plan à z constant d'un repère géographique) dans une représentation maillée à l'échelle fine d'une distribution de tailles de pores. On peut observer sur la figure 2 une grande variabilité de la taille (rayon R) des pores sur des distances très courtes (les dimensions de la représentation maillée est de 20 m dans la direction X, et de 20m dans la direction Y). On a choisi ici 100 nm pour délimiter les milieux petits pores et grands pores. On peut observer dans le tableau 1 que les pores de plus grandes tailles (de rayons supérieurs à 100 nm) représentent plus de 80% de la fraction volumique de l'ensemble des pores, alors que les pores de très petites tailles ne représentent que 20% de la fraction volumique de l'ensemble des pores. Les valeurs de porosité et de perméabilités de la matrice poreuse et du milieu fractures sont également données dans le tableau 1.

[Table 1]

| Milieu | Rayon de pore (nm) | Porosité | Perméabilité | Fraction volumique de pore (%) |
|--------|--------------------|----------|--------------|-------------------------------|
| Matrice | 2-100 | 0.036 |  | 20 |
|  | >100 | 0.1 | 100 nD | 80 |
| Fracture |  | 0.001 | 10D |  |

Le tableau 2 présente les paramètres d'écoulement du modèle de porosité après calage réalisé selon la mise en œuvre très préférée décrite ci-dessus. La perméabilité du milieu grands pores après calage est de 60nD, ce qui implique que celle du milieu petits pores est de 40 nD, ce qui revient à 100nD pour le milieu matriciel en général. Par ailleurs, l'écoulement inter grands pores est plus important que inter petits pores car les pores de rayons supérieurs à 100 nm représentent 80% du volume.

[Table 2]

| Rayon de pore équivalent du milieu petits pores (nm) | Perméabilité équivalente du milieu grands pores (nD) | Paramètre $\alpha$ |
|---|---|---|
| 6 | 60 | 0.08 |

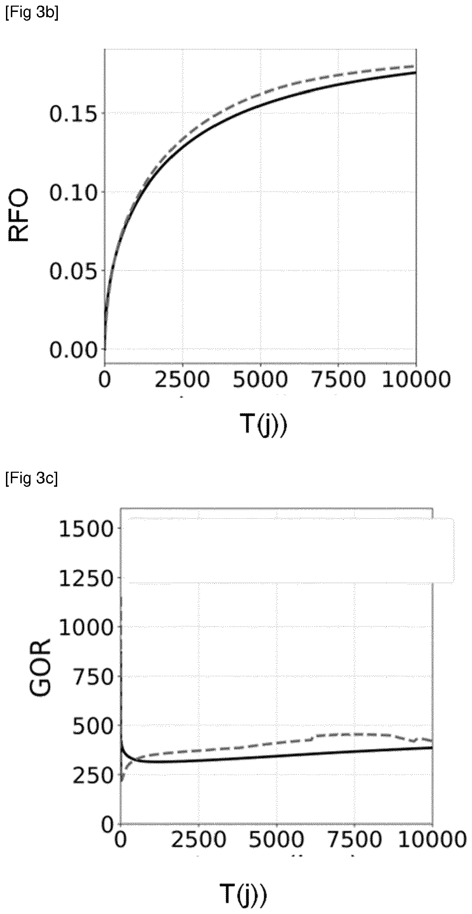

Les figures 3a, 3b, et 3c comparent des courbes de production de référence (courbes en pointillés) avec des courbes de production obtenues avec le modèle de porosité (courbes en trait plein) selon l'invention après calage appliqué tel que décrit dans le mode de réalisation préféré ci-dessus. Plus particulièrement, les figures 3a, 3b, et 3c présentent respectivement le facteur de récupération gaz (RFG), le facteur de récupération huile (RFO) et le rapport gaz-huile (GOR, pour Gas-Oil ratio) en fonction du temps T (en jours) obtenus à l'échelle fine (courbes en pointillés) et avec le modèle de porosité (courbes en trait plein). On peut observer sur ces figures que les courbes de production obtenues sur le modèle triple porosité selon l'invention après calage sont très proches des résultats de référence issus de la simulation à l'échelle fine. Le calage réalisé sur les trois paramètres d'écoulement que sont la dimension équivalente des pores du milieu petits pores, la perméabilité équivalente du milieu petits pores (ou de manière équivalente la perméabilité équivalente du milieu grands pores) et le paramètre $\alpha$ représentatif de la proportionnalité de la transmissibilité du milieu grands pores au milieu petits pores est donc particulièrement performant.

A l'issue de cette étape, appliquée pour chacune des classes de distribution de tailles de pores déterminées à l'issue de l'étape 1, on obtient un modèle de porosité calé pour chacune des classes de distribution de tailles de pores.

**4) Simulation d'écoulement au moyen des modèles de porosité déterminés pour chacune des classes de distribution de tailles de pores**

Au cours de cette étape, il s'agit de simuler les écoulements dans le réservoir géologique au moyen d'un simulateur d'écoulement selon l'invention et des modèles de porosité déterminés et calés pour chacune des classes de distribution de tailles de pores.

Selon l'invention, on attribue dans un premier temps un modèle de porosité en chacune des mailles de la représentation maillée. Etant donné qu'à l'issue de l'étape 1, une classe de distribution de tailles de pores a été attribuée à chacune des mailles de la représentation maillée du réservoir géologique, il suffit, pour une maille donnée, d'attribuer le modèle de porosité déterminé et calé pour la classe attribuée à cette maille.

Le simulateur d'écoulement, aussi appelé simulateur de réservoir, selon l'invention permet au moins :

a) la prise en compte d'un modèle triple porosité tel que décrit à l'étape 2 ci-dessus. Autrement dit, le simulateur d'écoulement selon l'invention est apte à considérer que chaque maille de la représentation maillée est découpée en blocs matriciels entourés de fractures, chaque bloc matriciel étant représenté par un milieu petits pores et un milieu grands pores. Plus précisément, selon l'invention, le simulateur d'écoulement selon l'invention implémente les modèles de porosité calés et attribués en chacune des mailles de la représentation maillée représentative du réservoir géologique étudié tel que décrits aux étapes 2 et 3 ci-dessus ;

b) la prise en compte pour le milieu petits pores d'une équation d'état thermodynamique prenant en compte la dimension équivalente des pores du milieu petits pores.

De manière générale, un calcul d'un flash thermodynamique, réalisé au moyen d'une équation d'état thermodynamique, est un calcul de l'équilibre liquide/vapeur d'un fluide pour une pression et une température donnée. Les données d'entrée d'un flash thermodynamique sont les fractions molaires $z_i$ des composants du mélange, la pression et la température. Les données de sorties sont les fractions molaires liquide $x_i$ et vapeur $y_i$ de chaque composants $i$ ainsi que les masse volumiques liquide et vapeur. L'équation d'état thermodynamique selon un mode de réalisation de l'invention est basée sur les équations de Rachford Rice et l'équation d'état de Peng Robinson. L'équation de Rachford Rice permet à partir de la fraction molaire $z_i$ et des coefficients d'équilibre des composants du mélange $K_i$ de calculer la fraction molaire vapeur V selon une formule du type :

[Math 1]

$$\sum_i \frac{(K_i - 1)z_i}{1 + V(K_i - 1)} = 0$$

L'équation de Peng Robinson est une expression analytique classique qui permet de relier la pression P, la température T et le volume molaire V. Elle permet également de calculer les coefficients de fugacités de chaque composé à l'état liquide et vapeur.

L'état d'équilibre liquide/vapeur est défini lorsque la fugacité de chaque composant du mélange à l'état liquide est égale à l'état vapeur.

De manière générale, un algorithme de flash thermodynamique optimise la valeur du coefficient d'équilibre Ki jusqu'à l'équilibre thermodynamique et donc l'égalité des fugacités liquide et vapeur de chaque composants.

Plus précisément, les étapes d'un algorithme de flash thermodynamique sont les suivantes. Le coefficient d'équilibre $K_i$ initial est calculé à partir de la solution analytique de Wilson. L'équation de Rachford Rice est résolue afin d'obtenir L et donc xi et yi qui permettent de calculer les coefficient de l'équation cubique de Peng Robinson. Un nouveau Ki peut donc être calculé à partir des coefficient de fugacité. Si l'égalité des fugacité n'est pas respecté, ce nouveau coefficient Ki est utilisé dans l'équation de Rachford Rice et l'algorithme recommence les mêmes étapes jusqu'à obtenir égalité des fugacités liquide/vapeur de chaque composant et donc d'atteindre l'équilibre thermodynamique.

Selon une mise en œuvre de l'invention, pour la pression critique, on peut utiliser la corrélation décrite dans le document (Meyra et al., 2005) pour chaque composants, et où la distance entre particules est telle que décrite dans (Bird et al., 2007). Selon une mise en œuvre de l'invention, pour la température critique, on peut utiliser la corrélation décrite dans (Jin et al, 2013).

Selon une mise en œuvre de l'invention, l'algorithme de flash thermodynamique est inchangé par rapport à un algorithme de flash thermodynamique selon l'art antérieur, mais c'est son utilisation qui est modifiée, les valeurs d'entrée de pression et température critique étant fonction du rayon de pores du milieu petits pores.

Selon l'invention, on prend ainsi en compte la fraction de pores dont les propriétés thermodynamiques et le point de bulle sont modifiés par la forte interaction fluide/pore dans le milieux petits pores.

c) des échanges de fluide se déroulant de la manière suivante exclusivement : des échanges de fluide du milieu grands pores vers le milieu petits pores, et des échanges de fluide du milieu petits pores vers le milieu fracture. Ainsi, une telle description des échanges de fluide exclus des échanges de fluide directement entre le milieux grands pores et le milieu fracture, contrairement au simulateur implémentant un modèle triple porosité tel que décrit dans (Alfi et al. 2017). De plus, le simulateur selon l'invention prend en compte la pression capillaire fonction de la saturation en gaz pour le calcul de l'écoulement.

Selon une mise en œuvre de l'invention, le simulateur d'écoulement utilisé pour la mise en œuvre du procédé selon l'invention implémente des équations de conservation de la masse telle que décrite par un système d'équations du type :

[Math 2]

$$
\begin{cases}
\dfrac{\partial}{\partial t}\left[\varepsilon^L \sum_p \rho_p^L C_{kp}^L S_p^L\right] + div\left[\sum_p \rho_p^L C_{kp}^L \overrightarrow{u_p^L}\right] + F_{kp}^{LS} = 0 \\[4mm]
\dfrac{\partial}{\partial t}\left[\varepsilon^S \sum_p \rho_p^S C_{kp}^S S_p^S\right] + div\left[\sum_p \rho_p^S C_{kp}^S \overrightarrow{u_p^S}\right] + F_{kp}^{Sf} - F_{kp}^{LS} = 0 \\[4mm]
\dfrac{\partial}{\partial t}\left[\varepsilon^f \sum_p \rho_p^f C_{kp}^f S_p^f\right] + div\left[\sum_p \rho_p^f C_{kp}^f \overrightarrow{u_p^f}\right] + \sum_p \rho_p^f C_{kp}^f Q_p^f - F_{kp}^{Sf} = 0
\end{cases}
$$

dans lequel les termes de ce système sont définis de la manière suivante :

exposant L : grands pores
exposant S : petits pores
exposant f : fracture
p : phase
k : composé du fluide
ε : porosité

$S_p$ : saturation de la phase p

$C_{kp}$ : fraction molaire du compose k dans la phase p $\rho_p$ : masse volumique de la phase p

F : flux, avec l'exposant 'Sf' signifiant 'petits pores' vers 'fractures', l'exposant 'LS' signifiant 'grands pores' vers 'petits pores'

u : vitesse obtenue par la loi de Darcy.

Un tel système décrit les termes d'accumulation et de flux convectifs dans chaque milieux ainsi que les flux d'échange entre milieux. Selon cette mise en œuvre de l'invention, le terme puits est négligé dans les milieux petits pores et grands pores car on considère que l'essentiel de la production s'effectue à travers les fractures. Les termes de flux d'échange entre milieux correspondent aux flux des grands pores vers les petits pores et des petits pores vers les fractures. La pression capillaire fonction de la saturation en gaz dans le milieu petits pores intervient dans la vitesse de Darcy.

Selon l'invention, avec un écoulement des grands pores à la fracture en passant par les petits pores, qui plus est en imposant une pression capillaire uniquement dans les petits pores, on prend en compte le phénomène de blocage capillaire du gaz.

d) les échanges de fluide se font exclusivement entre les milieux fracture entre deux mailles adjacentes du réservoir géologique. Autrement dit, il n'y a pas d'échanges de fluide entre les milieux petits pores et/ou grands pores d'une première maille et les milieu petits pores et/ou grands pores d'une deuxième maille qui serait connexe à la première maille. Ou autrement dit encore, les échanges ne se font d'une maille à une autre que via les fractures.

Selon l'invention, on simule les écoulements du fluide dans le réservoir géologique étudié au moyen du simulateur selon l'invention tel que décrit ci-dessus, de la représentation maillée de ce réservoir géologique et des modèles de porosités déterminés, calés et attribués en chacune des mailles de cette représentation maillée.

A l'issue de cette étape, on obtient par exemple des courbes de production du fluide, telles que des courbes de facteur de récupération gaz, des courbes de récupération d'huile, un ratio huile/gaz.

Selon une mise en œuvre de l'invention, on utilise en outre pour le simulateur selon l'invention une discrétisation de type couronnes emboîtées pour les deux milieux représentatifs de la matrice poreuse, c'est-à-dire pour le milieu petits pores et pour le milieu grand pores. Avantageusement, on utilise la discrétisation selon le modèle dit "modèle MINC" (Multiple Interacting Continua), décrite dans le document (Pruess, 1985). Plus précisément, selon cette mise en œuvre de l'invention, afin de prendre en compte le régime transitoire très lent entre matrice et fracture et les échanges internes entre petits pores et grands pores, les milieux matriciels petits pores et grands pores sont subdivisés en mailles MINC. L'écoulement à l'intérieur des grands pores et à l'intérieur des petits pores est considéré comme parallèle ; la perméabilité de la matrice est donc la somme des perméabilités des milieux petits pores et grands pores. Le flux entre le milieu petits pores et le milieu grands pores est similaire à celui d'un modèle double milieux entre la maille fracture et la première maille externe MINC du milieu petits pores. Les flux internes dans les grands pores et les petits pores suivent un modèle MINC. Selon une mise en œuvre de l'invention, le flux entre le milieu grands pores et le milieu petits pores suit un modèle MINC particulier, selon lequel la surface d'échange entre grands pores et petits pores est la couronne au milieu de deux mailles MINC matricielles et la distance d'échange comme la taille d'une maille MINC matricielle. La transmissibilité entre le milieu grands pores et le milieu petits pores est proportionnelle à cette surface et inversement proportionnelle à cette distance. Selon cette mise en œuvre de l'invention, un des paramètres d'écoulement du modèle de porosité peut être un paramètre $\alpha$ représentatif de la proportionnalité de la transmissibilité du milieu grands pores au milieu petits pores.

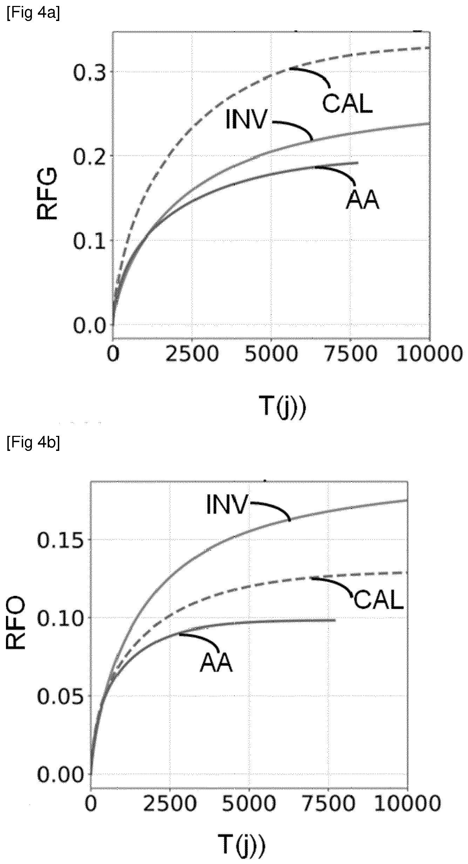

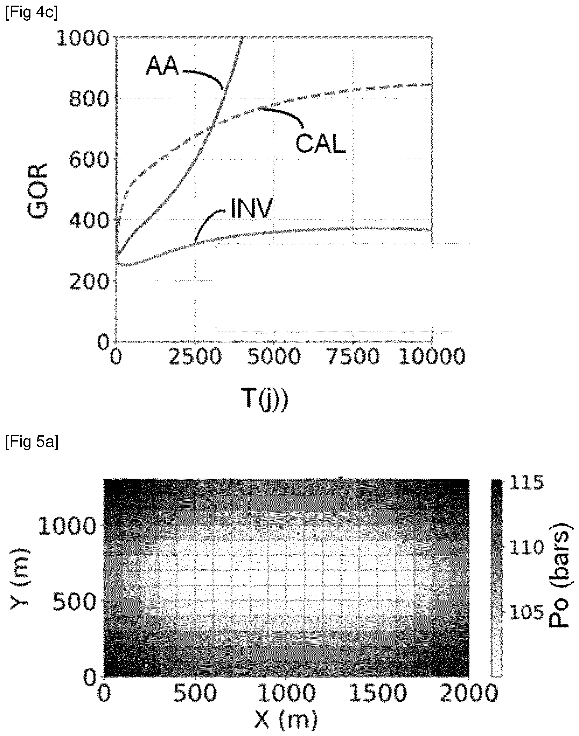

A titre illustratif, les figures 4a, 4b, et 4c présentent des courbes de production (facteur de récupération gaz (RFG), facteur de récupération huile (RFO) et rapport gaz-huile (GOR) en fonction du temps T (en jours), obtenus au moyen procédé selon l'invention (INV) appliqué au réservoir géologique présentant les distributions de tailles de pores telles que présentées en figure 1b. La tendance de production vis-à-vis du fluide non confiné suit les observations faites en maillage fin et en observation in situ (CAL). Le confinement augmente la production en huile et diminue la production en gaz et le GOR. A titre de comparaison ces mêmes courbes obtenues avec un modèle double porosité selon l'art antérieur (AA), confiné avec une taille de rayon correspondant au paramètre de calage a été tracé. Ce modèle couramment utilisé dans la littérature a un comportement vis-à-vis du fluide non confiné qui n'est pas du tout conforme aux observations.

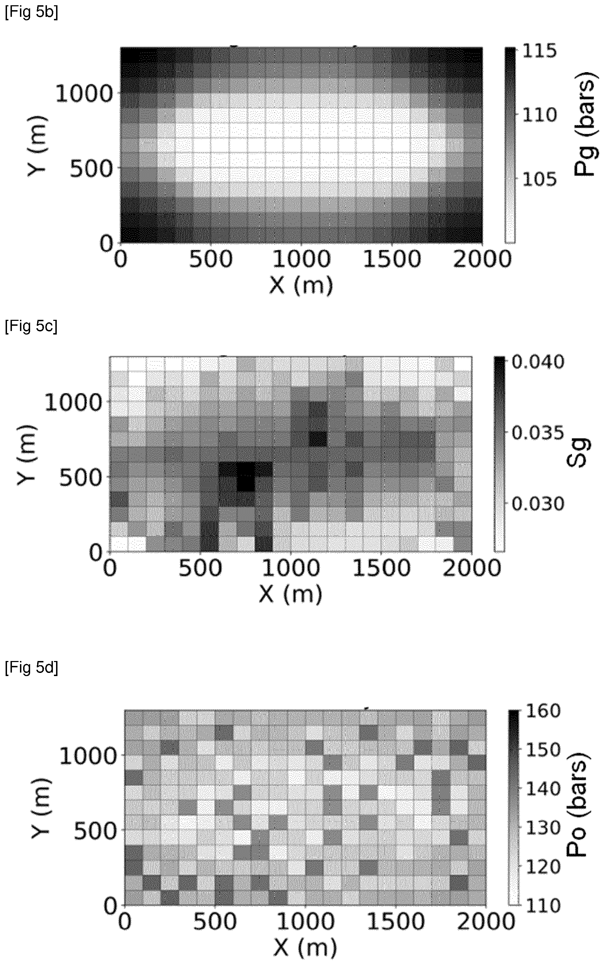

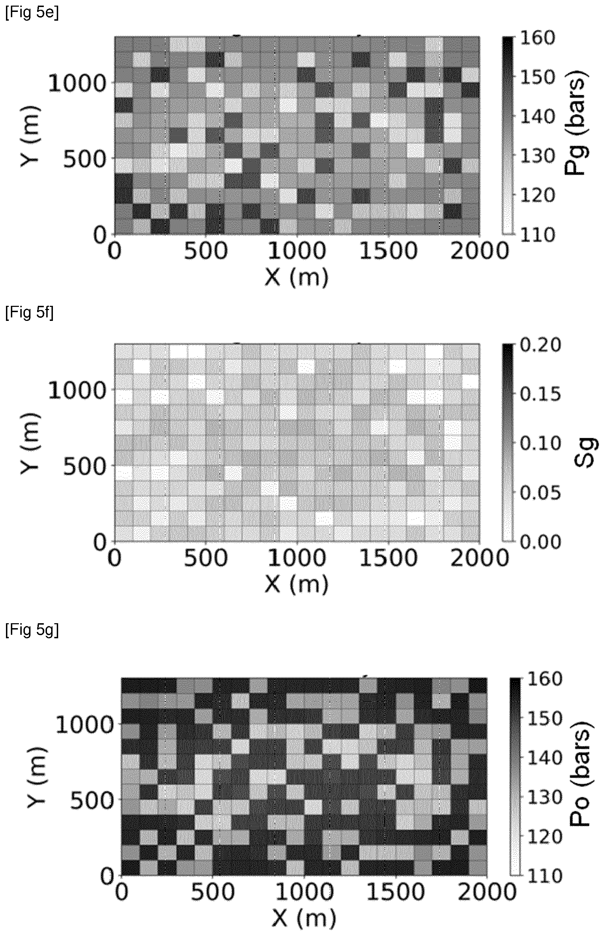

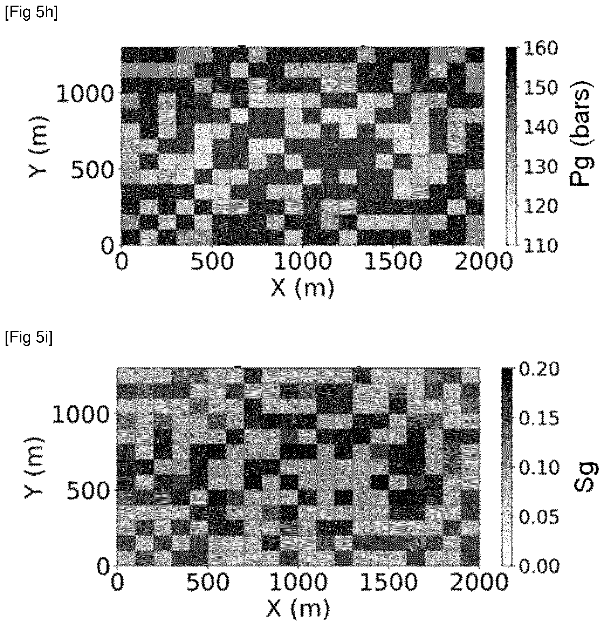

Par ailleurs, les figures 5a à 5i illustrent, pour un temps donné et en tout point d'une section à z constant dans le réservoir géologique étudié, des champs de pression huile (Po), des champs de pression gaz (Pg), des saturation en gaz (Sg) pour chacun des milieux du modèle triple porosité (figures 5a à 5c pour le milieu fractures, figures 5d à 5f pour le milieu petits pores et figures 5g à 5i pour le milieu grands pores). On peut observer sur ces figures que la saturation en gaz est plus importante dans les grand pores que dans les petits pores car le point de bulle est diminué dans les petits pores et à cause du blocage capillaire exercé dans les petits pore dont la pression capillaire huile/gaz n'est pas nulle. En effet, la pression huile gaz est similaire dans les grands pores et est différente dans les petits pores.

Ainsi, la présente invention selon son premier aspect permet une simulation plus précise des écoulements d'un fluide dans un réservoir géologique fracturé et présentant une taille de pores hétérogène. Ces avantages sont obtenus grâce à un simulateur d'écoulement implémentant un modèle triple porosité découpant le milieu matriciel en un milieu grands pores et un milieu petits pores, et permettant la prise en compte du régime transitoire très lent entre matrice poreuse et fractures, via notamment une thermodynamique dépendant de la taille des pores ainsi que la prise en compte d'hétérogénéités de pression capillaire.

Il est bien clair que le procédé selon l'invention comprend des étapes mises en œuvre au moyen d'un équipement (par exemple un poste de travail informatique) comprenant des moyens de traitement des données (un processeur) et des moyens de stockage de données (une mémoire, en particulier un disque dur), ainsi qu'une interface d'entrée et de sortie pour saisir des données et restituer les résultats du procédé.

En particulier, les moyens de traitement de données sont configurés pour mettre en œuvre la simulation des écoulements au sein du réservoir géologique étudié, au moyen d'un simulateur d'écoulement selon l'invention tel que décrit ci-dessus.

En outre, l'invention concerne un produit programme d'ordinateur téléchargeable depuis un réseau de communication et/ou enregistré sur un support lisible par ordinateur et/ou exécutable par un processeur, comprenant des instructions de code de programme pour la mise en œuvre du procédé tel que décrit précédemment, lorsque ledit programme est exécuté sur un ordinateur.

**5) Exploitation du fluide contenu dans le réservoir géologique**

Cette étape est mise en œuvre dans le cadre du procédé selon un deuxième aspect de l'invention, et qui concerne un procédé pour exploiter un fluide contenu dans le réservoir géologique étudié. Le fluide d'intérêt comprend des hydrocarbures, sous la forme d'une phase huile et d'une phase gazeuse.

Au cours de cette étape, il s'agit de déterminer au moins un schéma d'exploitation des hydrocarbures contenus dans le réservoir géologique étudié. De manière générale, un schéma d'exploitation comprend un nombre, une géométrie et une implantation (position et espacement) des puits injecteurs et producteurs à forer dans le réservoir étudié et à équiper. Un schéma d'exploitation peut en outre comprendre un type de récupération assistée des hydrocarbures contenus dans le réservoir, telle qu'une récupération au moyen de l'injection d'une solution comprenant un ou des polymères, de la mousse de $CO_2$, etc). Un schéma d'exploitation d'un réservoir d'hydrocarbures optimal doit par exemple permettre un fort taux de récupération des hydrocarbures piégés dans le réservoir géologique, sur une longue durée d'exploitation, et nécessitant un nombre de puits limité. Autrement dit, le spécialiste prédéfinit des critères d'évaluation selon lesquels un schéma d'exploitation du fluide d'un réservoir géologique est considéré comme suffisamment performant pour être mis en œuvre sur le réservoir géologique étudié.

Selon l'invention, la détermination du schéma d'exploitation des hydrocarbures du réservoir géologique étudié est réalisée à l'aide du simulateur d'écoulement selon l'invention, implémentant notamment le modèle triple porosité tel que décrit à l'étape 2 ci-dessus, ainsi qu'un flash thermodynamique tel que décrit à l'étape 4 ci-dessus et implémentant des écoulements tels que décrits à l'étape 4 entre les milieux du modèle triple porosité. Un tel simulateur de réservoir peut être établi à partir du simulateur d'écoulement PumaFlow® (IFP Energies nouvelles, France), que l'on modifie de manière à inclure les caractéristiques telles que décrites à l'étape 4 ci-dessus.

De manière générale, à tout instant t de la simulation, le simulateur d'écoulement résout l'ensemble des équations d'écoulement propres à chaque maille et délivre des valeurs solutions des inconnues (saturations, pressions, concentrations, température,...) prédites à cet instant t. De cette résolution, découle la connaissance des quantités d'huile produites et de l'état du gisement (distribution des pressions, saturations, etc...) à l'instant considéré. Au moyen des modèles de porosité déterminés, calés et attribués en chacune des mailles de la représentation maillée tel que décrit dans les étapes ci-dessus, le simulateur d'écoulement selon l'invention permet de prédire de manière fiable les productions notamment d'huile et de gaz pour un schéma d'exploitation donné.

Selon un mode de mise en œuvre de l'invention, on définit différents schémas d'exploitation du fluide contenu dans le réservoir géologique étudié et on estime, à l'aide du simulateur d'écoulement selon l'invention, au moins un critère, tel que la quantité d'hydrocarbures produit selon chacun des différents schémas d'exploitation, la courbe représentative de l'évolution de la production dans le temps au niveau de chacun des puits, le rapport huile sur gas (GOR) etc. Le schéma selon lequel les hydrocarbures contenus dans le réservoir sont réellement exploités peut alors correspondre à celui satisfaisant au moins un des critères d'évaluation des différentes schémas d'exploitation. Selon l'invention, on réalise une pluralité de simulations d'écoulement pour une pluralité d'implantations de puits injecteurs-producteurs, au moyen du simulateur selon l'invention et au moyen des modèles de porosité déterminés, calés et attribués en chacune des mailles de la représentation maillée, et on détermine le schéma d'exploitation selon lequel exploiter le fluide du réservoir géologique pour chacune des implantations et on sélectionne l'implantation satisfaisant au moins un des critères d'évaluation prédéfinis. Avantageusement, on peut en outre réaliser une pluralité de simulations d'écoulement pour une pluralité de type de récupération assistée, au moyen du simulateur selon l'invention et au moyen des modèles de porosité déterminés, calés et attribués en chacune des mailles de la représentation maillée, et on détermine le schéma d'exploitation selon lequel exploiter le fluide du réservoir géologique pour chacune des types de récupération assistée, et on sélectionne la récupération assistée satisfaisant au moins un des critères d'évaluation prédéfinis.

Puis, une fois le schéma d'exploitation déterminé, les hydrocarbures piégés dans le réservoir pétrolier sont exploités en fonction de ce schéma d'exploitation, notamment au moins en forant les puits injecteurs et producteurs du schéma d'exploitation ainsi déterminés, de manière à produire les hydrocarbures, et en installant les infrastructures de production nécessaires au développement de ce gisement. Dans le cas où le schéma d'exploitation a en outre été déterminé en estimant la production du réservoir associée à différents types de récupération assisté, on injecte dans le puis injecteur le ou les types d'additifs (polymère, tensio-actifs, mousse de $CO_2$) sélectionné tel que décrit ci-dessus.

Il est bien entendu que le schéma d'exploitation peut être évolutif sur la durée d'une exploitation des hydrocarbures d'un réservoir géologique, en fonction des connaissances relatives au réservoir acquises pendant l'exploitation, des améliorations dans les différents domaines techniques intervenant lors d'une exploitation d'un gisement d'hydrocarbures (améliorations dans le domaine du forage, de la récupération assistée par exemple).

## Revendications

1. Procédé mis en œuvre par ordinateur pour simuler des écoulements d'un fluide dans un réservoir géologique fracturé présentant une taille de pores hétérogène, ledit fluide comprenant une phase huile et une phase gazeuse, dans lequel on construit une première représentation maillée dudit réservoir à partir de mesures de propriétés relatives audit réservoir, **caractérisé en ce qu'**on applique au moins les étapes suivantes :

   A) à partir de mesures réalisées en laboratoire sur une pluralité d'échantillons provenant de différentes positions spatiales dans ledit réservoir géologique, on détermine des classes de distribution de tailles de pores, et on attribue une classe de distribution de tailles de pores en chacune des mailles de ladite première représentation maillée ;
   B) pour chacune desdites classes de distribution de tailles de pores, on construit un modèle de porosité représentatif de ladite distribution de tailles de pores, ledit modèle de porosité comprenant un premier milieu représentatif des pores de ladite distribution dont la dimension est comprise dans une première gamme, un deuxième milieu représentatif des pores de ladite distribution dont la dimension est comprise dans une deuxième gamme, et un troisième milieu représentatif desdites fractures de ladite distribution, lesdites dimensions desdits pores de ladite première gamme étant supérieures aux dimensions desdits pores de ladite deuxième gamme, ledit modèle de porosité étant en outre décrit par des paramètres d'écoulement pour chacun desdits milieux, lesdits paramètres d'écoulement comprenant au moins une dimension équivalente desdits pores dudit deuxième milieu et une pression capillaire fonction d'une saturation en ladite phase gazeuse dudit fluide dans ledit second milieu ;
   C) pour chacune desdites classes de distribution de tailles de pores, on cale au moins une partie desdits paramètres dudit modèle de porosité représentatif de ladite distribution de tailles de pores et on attribue ledit modèle de porosité calé en chacune des mailles de ladite première représentation maillée à laquelle est attribuée ladite classe ;

   et **en ce que** on simule lesdits écoulements dudit fluide dans ledit réservoir géologique au moyen de ladite première représentation maillée et d'un premier simulateur d'écoulement, ledit premier simulateur implémentant au moins :

   - lesdits modèles de porosité calés et attribués en chacune des mailles de ladite première représentation maillée ;
   - pour ledit second milieu, une équation d'état thermodynamique prenant en compte ladite dimension équivalente desdits pores dudit second milieu ;
   - au sein desdits modèles de porosité, des échanges dudit fluide entre ledit premier milieu et ledit deuxième milieu et entre ledit deuxième milieu et ledit troisième milieu d'un même modèle de porosité, en tenant compte de ladite pression capillaire fonction de ladite saturation en ladite phase gazeuse dudit fluide dans ledit second milieu ;
   - entre au moins deux desdits modèles de porosité, des échanges dudit fluide exclusivement entre lesdits troisièmes milieux desdits deux modèles de porosité.

2. Procédé selon la revendication 1, dans lequel ledit calage pour une desdites classes de distribution de pores est réalisé selon les étapes suivantes :

   a) on simule les écoulements au moyen dudit premier simulateur et dudit modèle de porosité représentatif de ladite classe de distribution de tailles de pores, ledit modèle de porosité étant défini par lesdits paramètres d'écoulement dudit modèle, et on obtient des courbes de production fonction desdits paramètres d'écoulement dudit modèle de porosité ;

b) on mesure les écarts entre lesdites courbes de production fonction desdits paramètres d'écoulement dudit modèle de porosité et des courbes de production de référence prédéterminées, et on corrige au moins une partie desdits paramètres d'écoulement dudit modèle de porosité de manière à minimiser lesdits écarts ;

c) on réitère les étapes a) et b) jusqu'à ce que lesdits écarts soient inférieurs à un seuil prédéfini, lesdits paramètres d'écoulement dudit modèle de porosité pour une itération de ladite réitération correspondant auxdits paramètres corrigés à une itération précédente de ladite réitération.

3. Procédé selon la revendication 2, dans lequel on détermine lesdites courbes de référence selon les étapes suivantes:

i) on construit une deuxième représentation maillée représentative de l'hétérogénéité de la taille des pores de ladite classe de distribution de tailles de pores, les dimensions des mailles de ladite deuxième représentation maillée étant déterminées de manière à rendre compte des effets induits par ladite taille des pores de ladite distribution ;

ii) on simule des écoulements au moyen de ladite deuxième représentation maillée et d'un deuxième simulateur d'écoulement implémentant au moins une équation d'état thermodynamique prenant en compte lesdites dimensions desdits pores de ladite classe de distribution de tailles pores, et on obtient des courbes de production dudit fluide relatives à ladite deuxième représentation maillée.

4. Procédé selon l'une des revendications 2 à 3, dans lequel ladite partie desdits paramètres d'écoulement corrigés comprend ladite dimension équivalente desdits pores dudit deuxième milieu, une perméabilité et une porosité équivalentes dudit deuxième milieu ou une perméabilité et une porosité équivalentes dudit premier milieu, et un paramètre représentatif de la proportionnalité de la transmissibilité dudit premier milieu audit deuxième milieu.

5. Procédé selon l'une des revendications précédentes, dans lequel lesdites mesures en laboratoire sont réalisées au moyen d'une méthode de porosimétrie au mercure et une méthode d'adsorption/désorption par azote.

6. Procédé selon l'une des revendications précédentes, dans lequel on discrétise lesdits premier milieu et deuxième milieux par une discrétisation de type couronnes emboîtées.

7. Produit programme d'ordinateur téléchargeable depuis un réseau de communication et/ou enregistré sur un support lisible par ordinateur et/ou exécutable par un processeur, comprenant des instructions de code de programme pour la mise en œuvre du procédé selon l'une des revendications précédentes, lorsque ledit programme est exécuté sur un ordinateur.

8. Procédé pour exploiter un fluide contenu dans un réservoir géologique fracturé présentant une taille de pores hétérogène, dans lequel on applique le procédé selon l'une quelconque des revendications 1 à 6, et dans lequel, à partir au moins de ladite simulation desdits écoulements dans ledit réservoir géologique, on détermine un schéma d'exploitation dudit réservoir géologique comprenant au moins une implantation d'au moins un puits injecteur et/ou d'au moins un puits producteur, et on exploite ledit fluide dudit réservoir géologique au moins en forant lesdits puits de ladite implantation et en les équipant d'infrastructures d'exploitation.

[Fig 1a]

[Fig 1b]

[Fig 2]

[Fig 3a]

[Fig 3b]

[Fig 3c]

[Fig 4a]

[Fig 4b]

[Fig 4c]

[Fig 5a]

[Fig 5b]

[Fig 5c]

[Fig 5d]

[Fig 5e]

[Fig 5f]

[Fig 5g]

[Fig 5h]

[Fig 5i]

Europäisches
Patentamt

European
Patent Office

Office européen
des brevets

**RAPPORT DE RECHERCHE EUROPEENNE**

Numéro de la demande

EP 20 16 5435

## DOCUMENTS CONSIDERES COMME PERTINENTS

| Catégorie | Citation du document avec indication, en cas de besoin, des parties pertinentes | Revendication concernée | CLASSEMENT DE LA DEMANDE (IPC) |
|---|---|---|---|
| X | Takuto Sakai ET AL: "Development of a Three-Dimensional, Three-Phase, Quadruple-Porosity/Quadruple-Permeability White Oil Type Simulator with Embedded Discrete Fracture Model for Predicting Shale Gas/Oil Flow Behavior", , 12 octobre 2017 (2017-10-12), pages 1-10, XP055667111, Extrait de l'Internet: URL:https://www.onepetro.org/conference-paper/SPWLA-JFES-2017-Q?sort=&start=0&q=development+of+a+three-dimensional%2C+three-phase%2C+quadruple-porosity&from_year=&peer_reviewed=&published_between=&fromSearchResults=true&to_year=&rows=25# [extrait le 2020-02-11] | 1-4,6,7 | INV. E21B43/00 G01V99/00 |
| Y | * figures 1-3, 6; tableaux 1-3 * | 5 | |
| A | | 8 | |
| | ----- | | |
| X | ALFI MASOUD ET AL: "Effect of pore sizes on composition distribution and enhance recovery from liquid shale-Molecular sieving in low permeability reservoirs", FUEL, IPC SCIENCE AND TECHNOLOGY PRESS, GUILDFORD, GB, vol. 235, 27 septembre 2018 (2018-09-27), pages 1555-1564, XP085500440, ISSN: 0016-2361, DOI: 10.1016/J.FUEL.2018.08.063 | 1-7 | DOMAINES TECHNIQUES RECHERCHES (IPC) E21B G01V |
| Y | * abrégé * | 5 | |
| A | * alinéas [0001] - [0002], [0004], [0007]; figures 2, 4 * | 8 | |
| | ----- | | |

Le présent rapport a été établi pour toutes les revendications

| Lieu de la recherche | Date d'achèvement de la recherche | Examinateur |
|---|---|---|
| La Haye | 9 juillet 2020 | Brassart, P |

CATEGORIE DES DOCUMENTS CITES

X : particulièrement pertinent à lui seul
Y : particulièrement pertinent en combinaison avec un
    autre document de la même catégorie
A : arrière-plan technologique
O : divulgation non-écrite
P : document intercalaire

T : théorie ou principe à la base de l'invention
E : document de brevet antérieur, mais publié à la
    date de dépôt ou après cette date
D : cité dans la demande
L : cité pour d'autres raisons

........................................................

& : membre de la même famille, document correspondant

EPO FORM 1503 03.82 (P04C02)

**RÉFÉRENCES CITÉES DANS LA DESCRIPTION**

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

**Documents brevets cités dans la description**

- EP 3181804 A

- US 20170212276 A

**Littérature non-brevet citée dans la description**

- **ALFI, M. ; AN, C. ; CAO, Y. et al.** Pore Size Variability and Sieving Effect in Liquid Shale-A Multiple Permeability Approach and Eagle Ford Case Study. *SPE Reservoir Simulation Conference,* 20 Février 2017
- **E.P. BARRET ; L.G. JOYNER ; P.B. HALENDA.** *J. Am. Chem. Soc.,* 1951, vol. 73, 373
- **BIRD, ROBERT BYRON ; STEWART, WARREN E. ; LIGHTFOOT, EDWIN N.** Transport phenomena. John Wiley & Sons Inc, 2007
- **CHALMERS, GARETH R. ; BUSTIN, R. MARC ; POWER, LAN M.** Characterization of gas shale pore systems by porosimetry, pycnometry, surface area, and field emission scanning electron microscopy/transmission electron microscopy image analyses. Examples from the Barnett, Woodford, Haynesville, Marcellus, and Doig units. *AAPG Bulletin,* 2012, vol. 96 (6), 1099-1119
- **JIN, LUCHAO ; MA, YIXIN ; JAMILI, AHMAD.** Investigating The Effect of Pore Proximity on Phase Behavior And Fluid Properties in Shale Formations. *SPE Annual Technical Conférence and Exhibition,* 2013

- **KUILA, UTPALENDU ; PRASAD, MANIKA.** Understanding Pore-Structure And Permeability In Shales. *SPE Annual Technical Conférence and Exhibition,* 2011
- **KUILA, UTPALENDU ; PRASAD, MANIKA.** Specific surface area and pore-size distribution in clays and shales. *Geophysical Prospecting,* 2013, vol. 61 (2), 341-362
- **LE RAVALEC M ; NOETINGER B ; HU LY.** The FFT moving average (FFT-MA) generator: an efficient tool for generating and conditioning Gaussian simulations. *Math Geol,* 2002, vol. 32 (6), 701-723
- **MEYRA, ARIEL G. ; ZARRAGOICOECHEA, GUILLERMO J. ; KUZ, VICTOR A.** Thermodynamic equations for a confined fluid at nanometric scale. *Fluid Phase Equilibria,* 2005, vol. 230 (1-2), 9-14
- **PRUESS, K.** A Practical Method for Modeling Fluid and Heat Flow in Fractured Porous Media. *Society of Petroleum Engineers,* 1985
- **WARREN, J.E. ; ROOT, P.J.** The Behavior of Naturally Fractured Reservoirs. *SPE Journal,* Septembre 1963, 245-255